# FASCICULE DE BREVET EUROPEEN

(11) **EP 3 096 800 B1**
(45) Date de publication et mention de la délivrance du brevet: **23.09.2020**
(21) Numéro de dépôt: 15701192.5
(22) Date de dépôt: 22.01.2015
(51) Int. Cl.: A61L 2/07, F17C 5/06, A61J 1/20, A61L 2/00, B67D 7/02

(54) **DISPOSITIF ET PROCÉDÉ POUR LE TRANSFERT D'UN PRODUIT STÉRILE ENTRE DEUX CONTENEURS**
VORRICHTUNG UND VERFAHREN ZUR ÜBERTRAGUNG EINES STERILEN PRODUKTS ZWISCHEN ZWEI BEHÄLTERN
DEVICE AND METHOD FOR TRANSFERRING A STERILE PRODUCT BETWEEN TWO CONTAINERS

(30) Priorité: 24.01.2014 FR 1450604
(43) Date de publication de la demande: 30.11.2016
(73) Titulaire: Pierre Fabre Dermo-Cosmétique, 92100 Boulogne-Billancourt (FR)
(72) Inventeur: DELAUNAY, Jean-Claude, F-34800 Clermont l'Herault (FR); SERRA, Laurent, F-34260 La Tour sur Orb (FR)
(74) Mandataire: Ipside
(86) Numéro de dépôt international: PCT/EP2015/051256
(87) Numéro de publication internationale: WO 2015/110531

(56) Documents cités:
- EP-A1- 1 260 235
- WO-A1-00/74735
- WO-A1-91/11203
- WO-A1-02/080990
- WO-A2-2009/045713
- GB-A- 2 398 244
- JP-A- H0 549 683
- US-A1- 2014 316 332

## Description

La présente invention concerne un dispositif pour le transfert en conditions aseptiques d'un produit, notamment d'un produit fluide, stérile, d'un premier conteneur dans un second conteneur, ainsi qu'un procédé de transfert mettant en œuvre un tel dispositif.

L'invention trouve notamment application dans les lignes de fabrication et/ou de conditionnement de produits stériles dans le domaine pharmaceutique, cosmétique, notamment dermo-cosmétique, vétérinaire ou encore agro-alimentaire. Ces produits peuvent aussi bien se présenter sous forme fluide, par exemple sous forme de gel, crème ou lait, et plus généralement sous forme de solutions ou de suspensions, que sous forme pulvérulente. Il peut notamment s'agir de médicaments injectables, collyres et pommades ophtalmiques, produits d'alimentation parentérale, compositions cosmétiques topiques, etc. Les conteneurs entre lesquels les produits stériles doivent être transférés peuvent en outre être de tout type, par exemple cuve à cuve, cuve à remplisseuse, etc.

Dans de tels domaines, les produits doivent répondre à des normes sévères de conservation vis-à-vis de la prolifération microbienne. Une des méthodes pour assurer cette conservation est d'inclure des conservateurs dans le produit. Ces conservateurs peuvent cependant être mal tolérés par l'utilisateur, et il est préférable de proposer des produits ne contenant pas de tels additifs, mais présentant tout de même une durée de conservation et une propreté microbienne adéquates. Cet objectif peut être atteint par la stérilisation des produits, et leur conservation dans des conditions propres à maintenir cette stérilité.

La stérilité d'un produit est ici définie de manière classique en elle-même, selon la norme EN 556 et la pharmacopée européenne en vigueur, comme la probabilité qu'un micro-organisme prolifère dans le produit. Typiquement, cette probabilité est inférieure à 10⁻⁶. Selon la norme ISO 13408 concernant la production stérile des produits de santé, le produit stérile doit avoir été traité par un procédé de stérilisation dont la valeur stérilisatrice F0 doit être supérieure à 15 minutes. Cette valeur F0, dont la méthode de détermination est définie par la pharmacopée européenne, en vigueur, donne un temps, exprimé en minutes, quantifiant l'effet létal de la chaleur humide à 121 °C sur les microorganismes viables. Un produit stérile au sens de la pharmacopée européenne a été soumis à un procédé de stérilisation dont la valeur stérilisatrice F0 est au moins égale à 15 minutes.

Il est fréquent, lors de leur fabrication ou de leur conditionnement, que les produits stériles doivent être transférés d'un premier conteneur stérile, qui les contient, dans un second conteneur également stérile, notamment pour leur stockage temporaire, ou encore pour leur répartition entre différentes lignes de conditionnement. Un tel transfert doit alors impérativement être réalisé dans des conditions permettant de maintenir la stérilité du produit lors de son parcours du premier conteneur au second conteneur.

De manière classique, les moyens utilisés pour un tel transfert sont des isolateurs munis de portes spéciales, dits DPTE (pour Dispositif de Porte pour Transfert Etanche), tels que celui décrit dans la demande de brevet EP 1 067 089, ou des moyens comme dans le brevet GB 2 398 244 B. Ces dispositifs imposent un environnement maîtrisé et surveillé sur le plan microbiologique, et le transfert est ainsi mis en œuvre dans une enceinte étanche dont l'atmosphère interne est maintenue stérile, impliquant notamment un renouvellement de son volume d'air interne et une filtration absolue. Un tel procédé de transfert, en conditions aseptiques, est long et contraignant à mettre en œuvre, car il requiert notamment d'importantes prises de précautions pour assurer le maintien de conditions de stérilité adéquates dans l'enceinte, de sorte à ce que rien n'y soit introduit qui puisse constituer une source de contamination microbienne du produit lors de son transfert d'un conteneur à l'autre. Le coût d'achat de tels systèmes, ainsi que les coûts de consommables, sont en outre élevés.

Des connecteurs aseptiques pour application à des solutions stériles ne nécessitant pas un environnement maîtrisé ont également été proposés par l'art antérieur. Cependant, ces connecteurs ne permettent pas le transfert de produits fluides de tous types, notamment des produits visqueux à un débit élevé, supérieur à 15 kg/heure. Ils ne sont donc nullement adaptés à un contexte de fabrication et conditionnement industriels de grandes quantités de produits stériles. Enfin, ils sont à usage unique.

La présente invention vise à remédier aux inconvénients des dispositifs et procédés pour le transfert d'un produit stérile, notamment d'un produit pharmaceutique ou cosmétique fluide, d'un premier conteneur à un autre, tels qu'ils sont proposés par l'art antérieur, et notamment aux inconvénients exposés ci-avant, en proposant un tel dispositif, et un procédé le mettant en œuvre, qui permettent de réaliser un tel transfert dans des conditions aseptiques, facilement et rapidement, et ce y compris dans un environnement non stérile, et quelles que soient les caractéristiques du produit à transférer, notamment sa viscosité. L'invention vise également à ce que ce dispositif soit réutilisable, après nettoyage et stérilisation, et à ce qu'il puisse assurer un transfert de produit avec des débits élevés.

A cet effet, il est proposé selon la présente invention un dispositif pour le transfert, en conditions aseptiques, d'un produit stérile d'un premier conteneur dans un second conteneur, ce dispositif comportant :
- un premier conteneur, contenant un produit stérile dans lequel la probabilité qu'un micro-organisme prolifère est inférieure à 10⁻⁶ selon la norme NF EN 556 équipé, de préférence en partie basse, de moyens de vidage comportant une première canalisation sur laquelle sont montées en série deux vannes d'ouverture / fermeture opérables indépendamment l'une de l'autre, dont une première vanne dite proximale disposée du côté du premier conteneur, et une deuxième vanne dite distale disposée du côté d'une extrémité libre opposée de la première canalisation,
- un second conteneur équipé, de préférence en partie basse, de moyens de remplissage comportant une seconde canalisation sur laquelle est montée une vanne d'ouverture / fermeture, de préférence sur laquelle sont montées en série deux vannes d'ouverture / fermeture opérables indépendamment l'une de l'autre, dont une première vanne dite proximale disposée du côté du second conteneur, et une deuxième vanne dite distale disposée du côté d'une extrémité libre opposée de la seconde canalisation,
- un conduit amovible de raccordement étanche de l'extrémité libre de la première canalisation et de l'extrémité libre de la seconde canalisation,
- et des moyens de connexion du conduit amovible à un système de stérilisation de son volume interne, opérables lorsque le conduit amovible raccorde de manière étanche l'extrémité libre de la première canalisation et l'extrémité libre de la seconde canalisation.

Selon l'invention, un procédé de transfert, en conditions aseptiques, d'un produit stérile du premier conteneur dans le second conteneur du dispositif selon l'invention, comprend les étapes successives de :
a/ remplissage du premier conteneur avec le produit stérile, dans une configuration dans laquelle au moins une vanne associée au premier conteneur, de préférence la vanne proximale, est fermée ;
b/ raccordement étanche par le conduit amovible de l'extrémité libre de la première canalisation et de l'extrémité libre de la seconde canalisation ;
c/ connexion du conduit amovible à un système de stérilisation de son volume interne ;
d/ stérilisation du volume interne du conduit amovible ;
e/ déconnexion du conduit amovible et du système de stérilisation de son volume interne ;
f/ ouverture de l'ensemble des vannes associées respectivement au premier conteneur et au second conteneur ;
g/ transfert du produit stérile du premier conteneur dans le second conteneur, via le conduit amovible ;
h/ et fermeture d'au moins une vanne associée au second conteneur.

Un tel dispositif, et son procédé de mise en œuvre, permettent avantageusement de transférer le produit stérile du premier conteneur dans le second conteneur dans des conditions aseptiques, ceci quel que soit l'environnement dans lequel se trouvent les conteneurs, y compris dans des environnements à conditions microbiologiques non maîtrisées. On entend dans la présente description, par conditions aseptiques, des conditions ne générant pas de contamination microbiologique du produit transféré, c'est-à-dire n'étant pas aptes à en dégrader la qualité microbiologique par une contamination externe.

Le procédé selon l'invention est en outre plus rapide et moins contraignant à mettre en œuvre que les procédés proposés par l'art antérieur. En effet, alors que l'art antérieur prévoit, pour mettre en œuvre le transfert du produit stérile d'un conteneur à l'autre, un maintien continu de la stérilité de l'environnement externe aux conteneurs, et notamment des moyens de transfert du produit entre les conteneurs, ce maintien nécessitant de nombreuses opérations spécifiques et fastidieuses, la présente invention prévoit au contraire une rupture temporaire de la stérilité, et la mise en œuvre d'un conduit amovible initialement non stérile. Ce conduit amovible est raccordé aux canalisations associées respectivement à chacun du premier conteneur et du second conteneur, sans rupture de la stérilité dans les conteneurs et dans les parties de canalisation qui leur sont respectivement contigües, puis il est avantageusement stérilisé *in situ,* de sorte à recréer un environnement stérile sur tout le parcours à emprunter par le produit stérile pour son transfert depuis le premier conteneur jusqu'au second conteneur. Il en résulte avantageusement un gain de temps, lié à l'absence de contraintes de maîtrise microbiologique environnementale.

Le dispositif et le procédé de transfert selon l'invention s'avèrent particulièrement utiles dans les contextes où il est nécessaire de déplacer des conteneurs contenant un produit stérile, par exemple pour fractionner un lot de production de produits stériles pour s'adapter à des besoins particuliers de conditionnement, ou pour déplacer le produit stérile d'une usine de production à une usine de conditionnement.

Selon des modes de mise en œuvre particuliers, l'invention répond en outre aux caractéristiques suivantes, mises en œuvre séparément ou en chacune de leurs combinaisons techniquement opérantes.

L'étape d/ de stérilisation du volume interne du conduit amovible peut être réalisée par toute méthode connue en elle-même. Dans des modes de mise en œuvre particuliers de l'invention, elle est réalisée par vapeur propre sous pression, au moyen d'un système de stérilisation classique, tel qu'un dispositif générateur de vapeur propre équipant fréquemment les sites de production et de conditionnement de produits stériles.

Préalablement à cette étape de stérilisation du volume interne du conduit amovible, le procédé selon l'invention prévoit avantageusement une étape de vérification de l'état de la vanne proximale et de la vanne distale associées au premier conteneur et, une étape de fermeture de celle desdites vannes qui pourrait ne pas être fermée. Cette étape s'avère notamment tout à fait avantageuse dans les modes de mise en œuvre particuliers de l'invention dans lesquels, lors de l'étape a/ de remplissage du premier conteneur par le produit stérile, la vanne proximale associée au premier conteneur est déjà fermée. En effet, il se forme alors, dans la partie de la première canalisation située entre la vanne proximale et la vanne distale, un volume vide stérile, qui isole, lors de la stérilisation du conduit amovible, le produit contenu dans le premier conteneur de la chaleur de la vapeur utilisée pour la stérilisation du conduit amovible. Ceci s'avère notamment particulièrement avantageux lorsque le produit contient un ou plusieurs constituants thermosensibles.

Dans des modes de réalisation particuliers de l'invention, le premier conteneur comporte, de préférence en partie haute, un moyen de raccordement étanche à un système de stérilisation de son volume interne. Ce moyen de raccordement étanche à un système de stérilisation est de préférence distinct de la première canalisation, entrant dans la constitution des moyens de vidage du premier conteneur. Le premier conteneur peut également comporter, de préférence également en partie haute, un moyen de raccordement étanche à un conduit de remplissage par le produit stérile, également de préférence distinct de la première canalisation. Ce moyen de raccordement étanche à un conduit de remplissage est de préférence confondu avec le moyen de raccordement étanche au système de stérilisation du volume interne du premier conteneur.

Le second conteneur peut également quant à lui comporter, de préférence en partie haute, un moyen de raccordement étanche à un système de stérilisation de son volume interne. Ce moyen de raccordement étanche à un système de stérilisation est de préférence distinct de la seconde canalisation, entrant dans la constitution des moyens de remplissage du second conteneur.

De telles caractéristiques permettent avantageusement de stériliser les volumes internes du premier conteneur et/ou du second conteneur *in situ,* notamment par un système de stérilisation par vapeur propre sous pression, par exemple un dispositif générateur de vapeur propre classique en lui-même. Un tel système de stérilisation par vapeur propre sous pression est de préférence le même que celui mis en œuvre pour la stérilisation du volume interne du conduit amovible.

De manière tout à fait avantageuse, lorsque le procédé selon l'invention comprend une étape de stérilisation du volume interne du premier conteneur et/ou une étape de stérilisation du volume interne du second conteneur, réalisée(s) par vapeur propre sous pression, cette étape est préférentiellement réalisée par introduction de vapeur propre sous pression dans le premier conteneur, respectivement le second conteneur, de préférence en partie haute dudit conteneur, respectivement du second conteneur, et évacuation des condensats par l'extrémité libre de la première canalisation, respectivement de la seconde canalisation.

Pour chacun du premier conteneur et du second conteneur, la canalisation associée remplit ainsi avantageusement une double fonction, liée d'une part à la stérilisation du volume interne du conteneur ainsi qu'à sa propre stérilisation et à celle des vannes, et d'autre part au transfert du produit stérile d'un conteneur à l'autre.

De la même manière, le moyen de raccordement étanche disposé en partie haute du premier conteneur peut également, dans des modes de réalisation particulièrement préférés de l'invention, remplir deux fonctions, d'une part pour la stérilisation du premier conteneur, et d'autre part pour son remplissage par le produit stérile.

Dans des modes de réalisation particuliers de l'invention, la première canalisation et/ou la seconde canalisation comporte(nt), entre la vanne distale et la vanne proximale associées, un point de purge.

La première canalisation et/ou la seconde canalisation présente(nt) en outre de préférence une section sensiblement uniforme sur toute leur longueur.

La stérilisation du second conteneur peut être effectuée préalablement à la stérilisation du conduit amovible. Dans ce cas, au moins une des vannes montées sur la seconde canalisation associée au second conteneur est fermée préalablement à la mise en œuvre de l'étape d/ de stérilisation du conduit amovible, de préférence la vanne proximale. Ainsi, dans des modes de mise en œuvre particuliers de l'invention, l'étape d/ de stérilisation du volume interne du conduit amovible est précédée d'une étape de fermeture d'au moins une vanne associée au second conteneur, préférentiellement la vanne proximale. Cette étape de fermeture est de préférence mise en œuvre avant l'étape b/ de raccordement étanche du conduit amovible à l'extrémité libre de la seconde canalisation. Préférentiellement, les deux vannes associées au second conteneur sont fermées préalablement au raccordement du conduit amovible à la seconde canalisation, de sorte à mieux sécuriser le dispositif en termes de stérilité.

L'invention n'exclut pas pour autant que la stérilisation du second conteneur et la stérilisation du conduit amovible puissent être réalisées simultanément, par exemple par introduction de vapeur propre sous pression dans le second conteneur, de préférence en partie haute du second conteneur, et évacuation des condensats au niveau du conduit amovible, à une extrémité de ce dernier éloignée du second conteneur, la vanne distale et la vanne proximale associées au second conteneur étant alors toutes deux ouvertes pour permettre à la vapeur d'eau sous pression de circuler depuis second conteneur, dans l'intégralité de la seconde canalisation, et dans le conduit amovible.

Le dispositif selon l'invention comporte en outre de préférence un tableau de connexion rapide des différents éléments constitutifs du dispositif, en particulier de la première canalisation et du premier conteneur, de la seconde canalisation et du second conteneur, et/ou du conduit amovible, à un système de stérilisation, en particulier un système du type par vapeur propre sous pression.

Préférentiellement, les vannes montées sur la première canalisation et/ou les vannes montées sur la seconde canalisation sont associées à des moyens de commande automatisés de leur ouverture / fermeture. Ces moyens sont en outre de préférence configurés de sorte à commander également le système de stérilisation associé au dispositif.

Le dispositif et le procédé de transfert selon l'invention permettent avantageusement de transférer tout type de produit stérile, notamment des produits de haute viscosité, à forts débits, supérieurs à 15 kg/heure, par un choix adéquat du diamètre des canalisations et du conduit de raccordement.

Ce dispositif est en outre entièrement réutilisable, puisqu'il suffit d'en nettoyer les éléments constitutifs, et le cas échéant de stériliser à nouveau les conteneurs, pour pouvoir mettre en œuvre un nouveau transfert de produit stérile. Ces opérations de nettoyage et de stérilisation peuvent avantageusement être réalisées *in situ,* sur le lieu même du transfert, par exemple au moyen d'un système de stérilisation par vapeur propre équipant de manière courante les sites de fabrication et conditionnement de produits stériles.

Le dispositif et le procédé selon l'invention sont peu coûteux respectivement à l'achat et en mise en œuvre.

Le transfert peut être réalisé facilement et rapidement, et de surcroit en zone dite non classée, c'est-à-dire sans contrainte microbiologique environnementale, dans le respect des normes de stérilité imposées pour les produits.

Selon une caractéristique avantageuse de l'invention, les deux vannes sont fermées et le volume interne de la partie de canalisation située entre les deux vannes est stérile, et constitue de ce fait une zone tampon entre l'intérieur et l'extérieur du conteneur. Les deux vannes forment avantageusement des barrières successives à la contamination microbienne pouvant survenir au niveau de l'extrémité libre de la canalisation située à l'opposé du conteneur, protégeant ainsi efficacement le produit stérile contenu dans le conteneur d'une telle contamination éventuelle.

L'invention sera maintenant plus précisément décrite dans le cadre de modes de réalisation préférés, qui n'en sont nullement limitatifs, représentés sur les figures 1 à 5, dans lesquelles :
- la figure 1 représente de manière schématique une partie des éléments constitutifs d'un dispositif selon un mode de réalisation de l'invention, dans une configuration correspondant à une étape préalable d'un procédé selon l'invention de transfert d'un produit stérile d'un premier conteneur dans un second conteneur, ladite étape consistant en la stérilisation du premier conteneur et du second conteneur ;
- la figure 2 montre le dispositif de la figure 1, dans une configuration à l'issue de ladite étape préalable ;
- la figure 3 montre le dispositif de la figure 1, dans une configuration correspondant à une étape ultérieure du procédé de transfert selon l'invention, consistant en la stérilisation d'un conduit amovible raccordant la première canalisation et la seconde canalisation ;
- la figure 4 montre le dispositif de la figure 3, dans une étape encore ultérieure du procédé de transfert selon l'invention, au cours de laquelle est réalisé le transfert du produit stérile du premier conteneur au second conteneur ;
- et la figure 5 illustre le dispositif de la figure 4, à l'issue de la mise en œuvre d'un procédé de transfert selon l'invention.

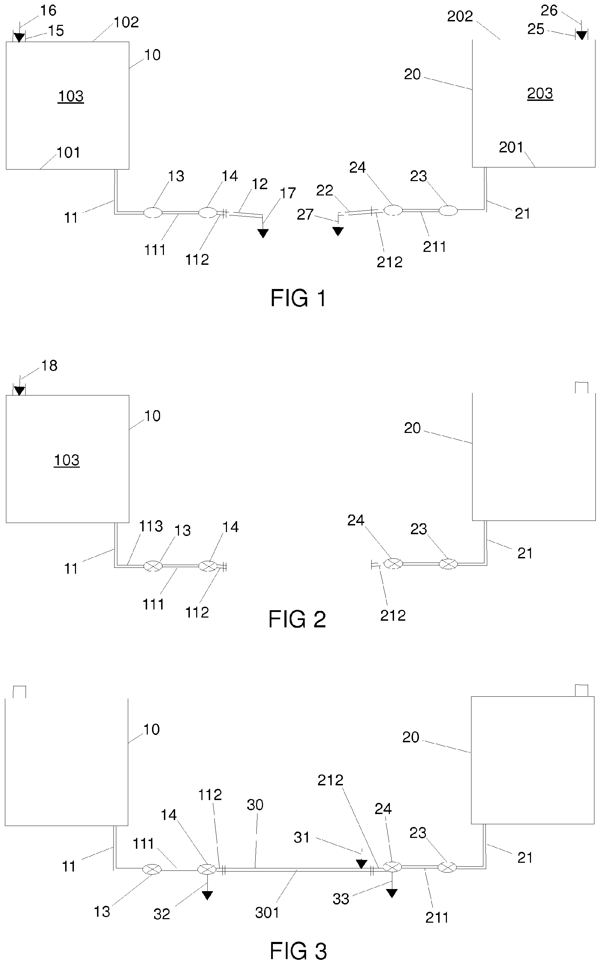

Un dispositif pour le transfert dans des conditions aseptiques d'un produit stérile d'un conteneur à un autre, selon un mode de réalisation particulier de la présente invention, est représenté de manière schématique, partiellement, sur la figure 1.

Ce dispositif comporte un premier conteneur 10 et un second conteneur 20. Ces conteneurs peuvent être de tout type couramment mis en œuvre dans les chaînes de fabrication / conditionnement de produits stériles. Il peut notamment s'agir de cuves de stockage et/ou de transfert de produits stériles fluides, formées en métal inoxydable. Ces cuves peuvent ou non présenter la même forme et le même volume interne.

Selon l'invention, le premier conteneur 10 est destiné à contenir initialement le produit stérile, et le second conteneur 20 est destiné à recevoir le produit stérile après son transfert, étant entendu que ces rôles respectifs sont interchangeables.

Le premier conteneur 10 est équipé d'une première canalisation 11 qui débouche dans sa paroi de fond 101. Sur cette première canalisation 11 sont montées deux vannes d'ouverture / fermeture de la canalisation, qui sont opérables indépendamment l'une de l'autre : une vanne dite proximale 13, disposée du côté du premier conteneur 10, et une vanne dite distale 14, disposée du côté de l'extrémité libre 112 de la première canalisation 11 opposée au premier conteneur 10.

La première canalisation 11 présente de préférence une section transversale sensiblement constante sur toute sa longueur. Sa partie disposée entre la vanne proximale 13 et la vanne distale 14 est dite partie intercalaire 111.

Le premier conteneur 10 comporte en outre, dans sa partie haute, par exemple, comme illustré sur la figure 1, au niveau de sa paroi supérieure 102, un moyen 15 de raccordement étanche à un système de stérilisation de son volume interne 103, par exemple à un dispositif générateur de vapeur propre, et à un conduit de remplissage. Ce moyen de raccordement est classique en lui-même.

Le second conteneur 20 présente des caractéristiques similaires à celles décrites ci-avant en référence au premier conteneur 10 : il est équipé d'une seconde canalisation 21 qui débouche dans sa paroi de fond 201. Sur cette seconde canalisation 21 sont montées deux vannes d'ouverture / fermeture de la canalisation, qui sont opérables indépendamment l'une de l'autre : une vanne dite proximale 23, disposée du côté du second conteneur 20, et une vanne dite distale 24, disposée du côté de l'extrémité libre 212 de la seconde canalisation 21 opposée au second conteneur 20.

La seconde canalisation 21 présente de préférence une section transversale constante sur toute sa longueur. Cette section peut être sensiblement identique à celle de la première canalisation 11. Sa partie disposée entre la vanne proximale 23 et la vanne distale 24 est dite partie intercalaire 211.

Le second conteneur 20 comporte en outre, dans sa partie haute, par exemple au niveau de sa paroi supérieure 202, un moyen 25 de raccordement étanche à un système de stérilisation de son volume interne 203, par exemple à un dispositif générateur de vapeur propre. Ce moyen de raccordement est classique en lui-même.

Par convention, dans les figures annexées à la présente description, la position de fermeture des vannes sera illustrée par une croix formée sur la vanne, alors que les vannes en position ouverte en seront dénuées.

Dans une étape préalable d'un procédé de transfert d'un produit stérile entre deux conteneurs selon un mode de mise en œuvre de l'invention, étape qui est illustrée sur la figure 1, le premier conteneur 10 et le second conteneur 20 sont soumis à un traitement de stérilisation. Ces opérations de stérilisation peuvent être réalisées simultanément ou successivement, y compris à des temps éloignés, au même endroit ou à des endroits différents, et au moyen du même système de stérilisation ou de systèmes différents.

La stérilisation est de préférence réalisée par vapeur propre sous pression, au moyen d'un dispositif générateur de vapeur propre classique en lui-même.

A cet effet, pour le premier conteneur 10 par exemple, le moyen de raccordement 15 disposé en partie haute du premier conteneur 10 est connecté, par exemple via un tableau de pontage prévu à cet effet, au système de stérilisation, non représenté sur les figures. L'extrémité libre 112 de la première canalisation 11 est connectée, par un conduit de récupération des condensats 12, au même tableau de pontage.

Les deux vannes respectivement proximale 13 et distale 14 sont ouvertes, et de la vapeur propre sous pression est introduite dans le premier conteneur 10, par le moyen de raccordement étanche 15, comme indiqué en 16 sur la figure 1. Cette vapeur propre remplit le premier conteneur 10, et s'en échappe via la première canalisation 11, par le conduit d'évacuation des condensats 12, comme indiqué en 17 sur la figure 1. Cette étape de traitement est mise en œuvre durant un temps suffisant pour assurer l'obtention d'un niveau de stérilité suffisant du volume interne 103 du premier conteneur 10, de la première canalisation 11 et des vannes proximale 13 et distale 14 associées.

A l'issue de cette étape, au moins une de ces vannes 13, 14 est fermée. De préférence, la vanne proximale 13 et la vanne distale 14 sont toutes deux fermées, de sorte à assurer que la partie intercalaire 111 de la première canalisation soit vide et stérile, constituant ainsi une zone tampon entre l'extérieur et l'intérieur du premier conteneur 10.

Les moyens de connexion du premier conteneur 10 au système de stérilisation sont déconnectés.

Les opérations de stérilisation peuvent être réalisées de manière similaire pour le second conteneur 20, la vapeur propre sous pression étant introduite dans le second conteneur 20 par le moyen de raccordement étanche 25, comme indiqué en 26 sur la figure 1, et les condensats s'échappant de la seconde canalisation 21 par un conduit d'évacuation 22, selon la direction indiquée en 27.

Le dispositif à l'issue de cette étape préalable est représenté de manière schématique sur la figure 2. Le premier conteneur 10 et le second conteneur 20 ont été déconnectés du ou des systèmes de stérilisation. Toutes les vannes sont fermées, et les volumes internes du premier conteneur 103 et du second conteneur 203, ainsi que de la première canalisation 11 et de la seconde canalisation 21, sont stériles.

Les parties intercalaires 111, 211 respectivement de la première canalisation 11 et de la seconde canalisation 21 constituent avantageusement des zones tampons qui protègent les volumes internes respectivement du premier conteneur 103 et du second conteneur 203 d'une éventuelle contamination microbiologique.

Une fois stérile, le premier conteneur 10 est rempli par le produit stérile à transférer. A cet effet, il est raccordé par le moyen de raccordement étanche 15 à un conduit de remplissage, non représenté sur la figure. Le produit stérile est ainsi introduit dans le premier conteneur 10, comme indiqué en 18 sur la figure 2. Il en remplit au moins partiellement le volume interne 103, ainsi que, par un phénomène de gravité, la partie de la première canalisation 11 disposée entre le premier conteneur 10 et la vanne proximale associée 13, dite partie proximale 113. La partie intercalaire 111 de la première canalisation 11 reste avantageusement vide et stérile.

L'étape suivante d'un procédé de transfert selon l'invention est illustrée sur la figure 3.

Le dispositif comporte un conduit amovible 30 pour le raccordement étanche de l'extrémité libre 112 de la première canalisation 11 et de l'extrémité libre 212 de la seconde canalisation 21. Ce conduit, initialement non stérile, est mis en place pour assurer ce raccordement étanche. Il présente de préférence une section transversale constante sur toute sa longueur. Cette section est de préférence sensiblement identique à celle de la première canalisation 11 et à celle de la seconde canalisation 21.

Le dispositif comporte en outre des moyens de connexion du conduit amovible 30 à un système de stérilisation, qui est classique en lui-même, et qui est de préférence identique à, ou au moins du même type que, celui mis en œuvre pour la stérilisation du premier conteneur 10 et/ou du second conteneur 20. De préférence, il s'agit d'un système de stérilisation par vapeur propre sous pression.

Ces moyens de connexion, non représentés sur les figures, peuvent être de tout type classique en lui-même, et ils sont opérables, de sorte à permettre la stérilisation du volume interne 301 du conduit amovible 30, lorsque ce dernier raccorde la première canalisation 11 et la seconde canalisation 21.

Ils sont configurés de sorte à assurer l'introduction de vapeur propre sous pression dans une première partie d'extrémité du conduit amovible 30, comme indiqué en 31 sur la figure 3, et l'évacuation des condensats au niveau d'une partie d'extrémité opposée, par exemple, mais non limitativement, comme indiqué en 32 sur la figure 3, au niveau de la vanne distale 14 associée à la première canalisation 11, par une troisième voie que cette vanne comporte à cet effet. Une partie des condensats peut également être évacuée au niveau de la vanne distale 24 associée à la seconde canalisation 21, comme indiqué en 33 sur la figure 3, de sorte à assurer que l'intégralité du volume interne 301 du conduit amovible 30 et des extrémités libres respectives de la première canalisation 11 et de la seconde canalisation 21 soit stérilisées, jusqu'aux vannes distales 14, 24. Ce traitement de stérilisation est poursuivi jusqu'à atteindre le niveau de stérilité adéquat.

Durant cette opération de stérilisation, la partie intercalaire 111 de la première canalisation 11 protège avantageusement le produit stérile contenu dans le premier conteneur 10, et dans la partie proximale 113 de la première canalisation 11, de la chaleur de la vapeur de stérilisation.

Concernant le second conteneur 20, durant cette opération, la vanne distale 24 peut être ouverte, de sorte à permettre un nouveau traitement de stérilisation de la partie intercalaire 211 de la seconde canalisation 21. Cette vanne distale 24 est de préférence fermée. Dans ce dernier cas, la vanne proximale 23 associée peut aussi bien être fermée, qu'ouverte. Préférentiellement, la vanne proximale 23 et la vanne distale 24 sont toutes deux fermées, de sorte à assurer le maintien de conditions stériles dans le second conteneur 20.

A l'issue de cette étape, l'ensemble du premier conteneur 10, de la première canalisation 11, du conduit amovible 30, de la seconde canalisation 21 et du second conteneur 20, c'est-à-dire l'intégralité du parcours à emprunter par le produit stérile pour son transfert du premier conteneur 10 au second conteneur 20, sont stériles.

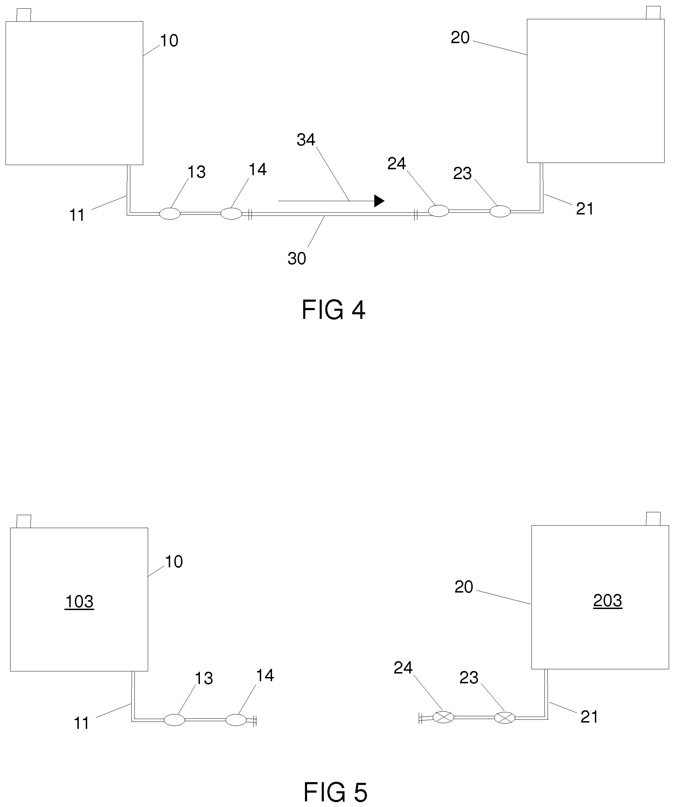

L'étape suivante, constituant le transfert proprement dit, est illustrée de manière schématique sur la figure 4.

L'ensemble des vannes, plus particulièrement la vanne proximale 13 et la vanne distale 14 associées au premier conteneur 10, et la vanne proximale 23 et la vanne distale 24 associées au second conteneur 20, sont ouvertes, pour opérer le transfert du produit stérile du premier conteneur 10 jusqu'au second conteneur 20, par l'intermédiaire de la première canalisation 11, du conduit amovible 30, puis de la seconde canalisation 21, comme indiqué en 34 sur la figure 4. Ce transfert s'effectue avantageusement en conditions aseptiques, en préservant la stérilité du produit transféré, sans qu'il soit besoin d'assurer une maîtrise microbiologique de l'environnement des conteneurs. Il permet de transférer facilement et rapidement tout type de produit, y compris les produits de viscosité élevée, et qui plus est au débit souhaité, ceci étant notamment permis par un choix pertinent du diamètre interne de la première canalisation 11, de la seconde canalisation 21 et du conduit amovible 30.

Lorsque le transfert du produit stérile dans le second conteneur 20 est terminé, la vanne distale 24 et la vanne proximale 23 montées sur la seconde canalisation 21 sont fermées, de sorte à isoler le volume interne 203 du second conteneur 20, contenant le produit stérile, de l'environnement extérieur.

Si l'intégralité du produit stérile initialement contenu dans le premier conteneur 10 n'a pas été transférée dans le second conteneur 20, la vanne distale 14 et la vanne proximale 13 montées sur la première canalisation 11 sont également fermées, de sorte à isoler le volume interne 103 du premier conteneur 10, contenant encore du produit stérile, de l'environnement extérieur

Le conduit amovible 30 est ensuite déconnecté de la seconde canalisation 21, ainsi que de la première canalisation 11, pour obtenir le dispositif dans sa configuration représentée de façon schématique sur la figure 5.

Le conduit amovible 30, ainsi que le premier conteneur 10 et la première canalisation 11, lorsqu'ils sont vides, peuvent être facilement nettoyés, notamment surplace, pour être réutilisés pour une autre opération de transfert ultérieure.

Le second conteneur 20, contenant le produit stérile, peut avantageusement être utilisé pour un transfert du produit stérile, en conditions aseptiques, dans un troisième conteneur, notamment par mise en œuvre d'un procédé conforme à l'invention, par exemple tel que décrit ci-avant. A cet effet, le second conteneur 20 joue alors le rôle d'un premier conteneur, ce qui est avantageusement permis par le fait qu'il présente des caractéristiques similaires au premier conteneur selon l'invention. Le procédé décrit ci-avant peut alors être appliqué à nouveau, à l'exception des étapes de stérilisation et de remplissage du premier conteneur, qui n'ont plus à être effectuées.

## Revendications

1. Dispositif pour le transfert en conditions aseptiques d'un produit stérile d'un premier conteneur (10) dans un second conteneur (20), comportant :
- un premier conteneur (10) contenant un produit stérile, dans lequel la probabilité qu'un micro-organisme prolifère est inférieure à 10⁻⁶ selon la norme NF EN 556, équipé de moyens de vidage comportant une première canalisation (11) sur laquelle sont montées en série deux vannes d'ouverture / fermeture (13, 14) opérables indépendamment l'une de l'autre, dont une première vanne dite proximale (13) disposée du côté dudit premier conteneur, et une deuxième vanne dite distale (14) disposée du côté d'une extrémité libre opposée (112) de ladite première canalisation,
- un second conteneur (20) équipé de moyens de remplissage comportant une seconde canalisation (21) sur laquelle sont montées en série deux vannes d'ouverture / fermeture (23, 24) opérables indépendamment l'une de l'autre, dont une première vanne dite proximale (23) disposée du côté dudit second conteneur, et une deuxième vanne dite distale (24) disposée du côté d'une extrémité libre opposée (212) de ladite seconde canalisation,
- un conduit amovible (30) de raccordement étanche de ladite extrémité libre de la première canalisation et de ladite extrémité libre de la seconde canalisation,
- et des moyens de connexion dudit conduit amovible à un système de stérilisation de son volume interne (301) opérables lorsque ledit conduit amovible raccorde de manière étanche ladite extrémité libre de la première canalisation et ladite extrémité libre de la seconde canalisation.

2. Dispositif selon la revendication 1, dans lequel le premier conteneur (10) comporte un moyen (15) de raccordement étanche à un système de stérilisation de son volume interne (103).

3. Dispositif selon la revendication 2, dans lequel le moyen (15) de raccordement étanche du premier conteneur (10) à un système de stérilisation de son volume interne (103) est distinct de la première canalisation (11).

4. Dispositif selon l'une quelconque des revendications 1 à 3, dans lequel le premier conteneur (10) comporte un moyen (15) de raccordement étanche à un conduit de remplissage, de préférence confondu avec ledit moyen de raccordement étanche à un système de stérilisation de son volume interne (103).

5. Dispositif selon l'une quelconque des revendications 1 à 4, dans lequel le second conteneur (20) comporte un moyen (25) de raccordement étanche à un système de stérilisation de son volume interne (203).

6. Dispositif selon la revendication 5, dans lequel le moyen (25) de raccordement étanche du second conteneur (20) à un système de stérilisation de son volume interne (203) est distinct de la seconde canalisation (21).

7. Procédé de transfert d'un produit stérile d'un premier conteneur (10) dans un second conteneur (20), au moyen d'un dispositif selon l'une quelconque des revendications 1 à 6, comprenant les étapes successives de :
a/ remplissage du premier conteneur (10) avec le produit stérile, dans une configuration dans laquelle au moins une vanne associée au premier conteneur (13, 14) est fermée ;
b/ raccordement étanche par ledit conduit amovible (30) de l'extrémité libre (112) de la première canalisation (11) et de l'extrémité libre (212) de la seconde canalisation (21) ;
c/ connexion dudit conduit amovible (30) à un système de stérilisation de son volume interne ;
d/ stérilisation du volume interne (301) du conduit amovible (30) ;
e/ déconnexion du conduit amovible (30) et du système de stérilisation de son volume interne ;
f/ ouverture de l'ensemble des vannes associées respectivement au premier conteneur (13, 14) et au second conteneur (23, 24) ;
g/ transfert du produit stérile du premier conteneur (10) dans le second conteneur (20), via le conduit amovible (30) ;
h/ et fermeture d'au moins une vanne associée au second conteneur (23, 24).

8. Procédé selon la revendication 7, selon lequel l'étape d/ de stérilisation du volume interne (301) du conduit amovible (30) est réalisée par vapeur propre sous pression.

9. Procédé selon l'une quelconque des revendications 7 à 8, selon lequel l'étape d/ de stérilisation du volume interne (301) du conduit amovible (30) est précédée d'une étape de fermeture d'au moins une vanne associée au second conteneur (23, 24), préférentiellement de la vanne proximale (23), ladite étape de fermeture étant de préférence mise en oeuvre avant l'étape b/ de raccordement étanche du conduit amovible (30) à l'extrémité libre (212) de la seconde canalisation (21).

10. Procédé selon l'une quelconque des revendications 7 à 9, comprenant une étape préalable de stérilisation du volume interne (203) du second conteneur (20).

11. Procédé selon l'une quelconque des revendications 7 à 10, comprenant une étape préalable de stérilisation du volume interne (103) du premier conteneur (10).

12. Procédé selon l'une quelconque des revendications 10 à 11, selon lequel la stérilisation du volume interne (203) du second conteneur (20) et/ou du volume interne (103) du premier conteneur (10) est réalisée par vapeur propre sous pression, de préférence au moyen du même système de stérilisation que le système de stérilisation mis en oeuvre pour la stérilisation du volume interne (301) du conduit amovible (30).

13. Procédé selon la revendication 12, selon lequel la stérilisation du volume interne (203) du second conteneur (20) est réalisée par introduction de vapeur propre sous pression dans le second conteneur et évacuation des condensats par l'extrémité libre (212) de la seconde canalisation (21), et/ou la stérilisation du volume interne (103) du premier conteneur (10) est réalisée par introduction de vapeur propre sous pression dans le premier conteneur et évacuation des condensats par l'extrémité libre (112) de la première canalisation (11).

## Patentansprüche

1. Vorrichtung zur Übertragung unter aseptischen Bedingungen eines sterilen Produkts von einem ersten Behälter (10) in einen zweiten Behälter (20), Folgendes beinhaltend:
- einen ersten Behälter (10), der ein steriles Produkt enthält, in dem die Wahrscheinlichkeit, dass sich ein Mikroorganismus stark vermehrt, geringer als 10⁻⁶ gemäß der Norm NF EN 556 ist, der mit Entleerungsmitteln ausgerüstet ist, die eine erste Kanalisation (11) beinhalten, in der in Reihe zwei Ventile zum Öffnen / Schließen (13, 14) montiert sind, die unabhängig voneinander betreibbar sind, darunter ein erstes, sogenanntes proximales (13) Ventil, das auf Seiten des ersten Behälters angeordnet ist, und ein zweites, sogenanntes distales Ventil (14), das auf Seiten eines entgegengesetzten freien Endes (112) der ersten Kanalisation angeordnet ist,
- einen zweiten Behälter (20), der mit Füllungsmitteln ausgerüstet ist, die eine zweite Kanalisation (21) beinhalten, in der in Reihe zwei Ventile zum Öffnen / Schließen (23, 24) montiert sind, die unabhängig voneinander betreibbar sind, darunter ein erstes, sogenanntes proximales (23) Ventil, das auf Seiten des zweiten Behälters angeordnet ist, und ein zweites, sogenanntes distales (24) Ventil, das auf Seiten eines entgegengesetzten freien Endes (212) der zweiten Kanalisation angeordnet ist,
- eine abnehmbare dichte Anschlussleitung (30) des freien Endes der ersten Kanalisation und des freien Endes der zweiten Kanalisation,
- und Verbindungsmittel der abnehmbaren Leitung mit einem Sterilisierungssystem ihres Innenvolumens (301), die betreibbar sind, wenn die abnehmbare Leitung in dichter Form das freie Ende der ersten Kanalisation und das freie Ende der zweiten Kanalisation anschließt.

2. Vorrichtung nach Anspruch 1, wobei der erste Behälter (10) ein Mittel (15) zum dichten Anschließen an ein Sterilisierungssystem seines Innenvolumens (103) beinhaltet.

3. Vorrichtung nach Anspruch 2, wobei das Mittel (15) zum dichten Anschließen des ersten Behälters (10) an ein Sterilisierungssystem seines Innenvolumens (103) von der ersten Kanalisation (11) verschieden ist.

4. Vorrichtung nach einem der Ansprüche 1 bis 3, wobei der erste Behälter (10) ein Mittel (15) zum dichten Anschließen an eine Füllungsleitung beinhaltet, das vorzugsweise mit dem Mittel zum dichten Anschließen an ein Sterilisierungssystem seines Innenvolumens (103) zusammengenommen ist.

5. Vorrichtung nach einem der Ansprüche 1 bis 4, wobei der zweite Behälter (20) ein Mittel (25) zum dichten Anschließen an ein Sterilisierungssystem seines Innenvolumens (203) beinhaltet.

6. Vorrichtung nach Anspruch 5, wobei das Mittel (25) zum dichten Anschließen des zweiten Behälters (20) an ein Sterilisierungssystem seines Innenvolumens (203) von der zweiten Kanalisation (21) verschieden ist.

7. Verfahren zur Übertragung eines sterilen Produkts von einem ersten Behälter (10) in einen zweiten Behälter (20), anhand einer Vorrichtung nach einem der Ansprüche 1 bis 6, die folgenden aufeinanderfolgenden Schritte umfassend:
a/ Füllen des ersten Behälters (10) mit dem sterilen Produkt, in einer Konfiguration, bei der mindestens ein dem ersten Behälter zugewiesenes Ventil (13, 14) geschlossen ist;
b/ dichtes Anschließen durch die abnehmbare Leitung (30) des freien Endes (112) der ersten Kanalisation (11) und des freien Endes (212) der zweiten Kanalisation (21);
c/ Verbinden der abnehmbaren Leitung (30) mit einem Sterilisierungssystem ihres Innenvolumens;
d/ Sterilisieren des Innenvolumens (301) der abnehmbaren Leitung (30);
e/ Trennen der abnehmbaren Leitung (30) und des Sterilisierungssystems ihres Innenvolumens;
f/ Öffnen der Gesamtheit der jeweils dem ersten Behälter (13, 14) und dem zweiten Behälter (23, 24) zugewiesenen Ventile;
g/ Übertragen des sterilen Produkts vom ersten Behälter (10) in den zweiten Behälter (20) über die abnehmbare Leitung (30);
h/ und Schließen mindestens eines dem zweiten Behälter zugewiesenen Ventils (23, 24).

8. Verfahren nach Anspruch 7, wobei der Schritt d/ zum Sterilisieren des Innenvolumens (301) der abnehmbaren Leitung (30) durch sauberen Dampf unter Druck realisiert wird.

9. Verfahren nach einem der Ansprüche 7 bis 8, wobei dem Schritt d/ zum Sterilisieren des Innenvolumens (301) der abnehmbaren Leitung (30) ein Schritt zum Schließen mindestens eines dem zweiten Behälter zugewiesenen Ventils (23, 24), vorzugsweise des proximalen Ventils (23) vorangeht, wobei der Schritt zum Schließen vorzugsweise vor dem Schritt b/ zum dichten Anschließen der abnehmbaren Leitung (30) an das freie Ende (212) der zweiten Kanalisation (21) umgesetzt wird.

10. Verfahren nach einem der Ansprüche 7 bis 9, einen vorangehenden Schritt zum Sterilisieren des Innenvolumens (203) des zweiten Behälters (20) umfassend.

11. Verfahren nach einem der Ansprüche 7 bis 10, einen vorangehenden Schritt zum Sterilisieren des Innenvolumens (103) des ersten Behälters (10) umfassend.

12. Verfahren nach einem der Ansprüche 10 bis 11, wobei die Sterilisierung des Innenvolumens (203) des zweiten Behälters (20) und/oder des Innenvolumens (103) des ersten Behälters (10) durch sauberen Dampf unter Druck, vorzugsweise anhand desselben Sterilisierungssystems realisiert wird, wie das Sterilisierungssystem, das für die Sterilisierung des Innenvolumens (301) der abnehmbaren Leitung (30) umgesetzt wird.

13. Verfahren nach Anspruch 12, wobei die Sterilisierung des Innenvolumens (203) des zweiten Behälters (20) durch Einleiten von sauberem Dampf unter Druck in den zweiten Behälter und Evakuieren der Kondensate durch das freie Ende (212) der zweiten Kanalisation (21) realisiert wird, und/oder die Sterilisierung des Innenvolumens (103) des zweiten Behälters (10) durch Einleiten von sauberem Dampf unter Druck in den ersten Behälter und Evakuieren der Kondensate durch das freie Ende (112) der ersten Kanalisation (11) realisiert wird.

## Claims

1. Device for transferring a sterile product in aseptic conditions from a first container (10) into a second container (20), comprising:
- a first container (10) containing a sterile product, wherein the probability of a microorganism proliferating is less than 10⁻⁶ according to standard NF EN 556, equipped with emptying means comprising a first pipe (11) on which there are fitted in series two opening/closure valves (13, 14) which can be operated independently from one another, comprising a first valve, called the proximal valve (13), arranged on the first container side, and a second valve, called the distal valve (14), arranged on the side of an opposite free end (112) of said first pipe;
- a second container (20) which is equipped with filling means comprising a second pipe (21) on which there are fitted in series two opening/closure valves (23, 24) which can be operated independently from one another, comprising a first valve, called the proximal valve (23), arranged on the second container side, and a second valve, called the distal valve (24), arranged on the side of an opposite free end (212) of said second pipe;
- a removable hose (30) for sealed connection of said free end of the first pipe and said free end of the second pipe; and
- means for connection of said removable hose to a system (301) for sterilization of its inner volume, which can be operated when said removable hose connects said free end of the first pipe and said free end of the second pipe in a sealed manner.

2. Device as claimed in claim 1, wherein the first container (10) comprises a means (15) for sealed connection to a system (103) for sterilization of its inner volume.

3. Device as claimed in claim 2, wherein the means (15) for sealed connection of the first container (10) to a system (103) for sterilization of its inner volume is distinct from the first pipe (11).

4. Device as claimed in any one of claims 1 to 3, wherein the first container (10) comprises a means (15) for sealed connection to a filling hose, which preferably coincides with said means for sealed connection to a system (103) for sterilization of its inner volume.

5. Device as claimed in any one of claims 1 to 4, wherein the second container (20) comprises a means (25) for sealed connection to a system (203) for sterilization of its inner volume.

6. Device as claimed in claim 5, wherein the means (25) for sealed connection of the second container (20) to a system (203) for sterilization of its inner volume is distinct from the second pipe (21).

7. Method for transferring a sterile product from a first container (10) into a second container (20) by means of a device according to any one of claims 1 to 6, comprising the successive steps of:
a/ filling the first container (10) with the sterile product, in a configuration in which at least one valve (13, 14) associated with the first container is closed;
b/ sealed connection by said removable hose (30) of the free end (112) of the first pipe (11) and of the free end (212) of the second pipe (21);
c/ connection of said removable hose (30) to a system for sterilization of its inner volume;
d/ sterilization of the inner volume (301) of the removable hose (30);
e/ disconnection of the removable hose (30) and of the system for sterilization of its inner volume;
f/ opening of all of the valves associated respectively with the first container (13, 14) and the second container (23, 24);
g/ transferring the sterile product from the first container (10) into the second container (20), via the removable hose (30); and
h/ closure of at least one valve associated with the second container (23, 24).

8. Method as claimed in claim 7, wherein the step d/ of sterilization of the inner volume (301) of the removable hose (30) is carried out by pressurized clean steam.

9. Method as claimed in either of claims 7 and 8, wherein the step d/ of sterilization of the inner volume (301) of the removable hose (30) is preceded by a step of closure of at least one valve associated with the second container (23, 24), preferably of the proximal valve (23), said step of closure preferably being implemented before the step b/ of sealed connection of the removable hose (30) at the free end (212) of the second pipe (21).

10. Method as claimed in any one of claims 7 to 9, comprising a preliminary step of sterilization of the inner volume (203) of the second container (20).

11. Method as claimed in any one of claims 7 to 10, comprising a preliminary step of sterilization of the inner volume (103) of the first container (10).

12. Method as claimed in either of claims 10 and 11, wherein the sterilization of the inner volume (203) of the second container (20) and/or of the inner volume (103) of the first container (10) is carried out by pressurized clean steam, preferably by means of the same sterilization system as the sterilization system implemented for the sterilization of the inner volume (301) of the removable hose (30).

13. Method as claimed in claim 12, wherein the sterilization of the inner volume (203) of the second container (20) is carried out by introduction of pressurized clean steam into the second container and discharge of the condensates via the free end (212) of the second pipe (21), and/or the sterilization of the inner volume (103) of the first container (10) is carried out by introduction of pressurized clean steam into the first container and discharge of the condensates via the free end (112) of the first pipe (11).
